# FASCICULE DE BREVET EUROPEEN

(11) **EP 3 033 124 B1**
(45) Date de publication et mention de la délivrance du brevet: **28.04.2021**
(21) Numéro de dépôt: 14750223.1
(22) Date de dépôt: 11.08.2014
(51) Int. Cl.: A61M 5/145, A61M 5/142, A61M 5/148

(54) **POMPE A LIQUIDES AUTONOME POUR VEHICULER UN FLUIDE VERS UN ETRE VIVANT**
SELBSTÄNDIGE FLÜSSIGKEITSPUMPE ZUR FÖRDERUNG EINER FLÜSSIGKEIT ZU EINEM PATIENTEN
INDEPENDENT LIQUID PUMP FOR THE PROMOTION OF A LIQUID TO A PATIENT

(30) Priorité: 13.08.2013 BE 201300541
(43) Date de publication de la demande: 22.06.2016
(73) Titulaire: Mahusaca SPRL, 4877 Olne (BE)
(72) Inventeur: PEETERS, Thierry, 4877 Olne (BE); DURY, François, 4031 Angleur (BE); RENARD, Philippe, 4190 Xhoris (BE); GANGOLF, Dimitri, 4690 Thimister (BE)
(74) Mandataire: ABYOO
(86) Numéro de dépôt international: PCT/EP2014/067151
(87) Numéro de publication internationale: WO 2015/022287

(56) Documents cités:
- EP-A2- 1 964 585
- WO-A1-97/34651
- WO-A1-2008/080442
- WO-A1-2011/075104
- WO-A2-02/49707
- WO-A2-2004/024211
- DE-A1- 10 058 121

## Description

### Domaine de l'invention

L'invention se rapporte à un appareil autonome pour véhiculer un fluide vers un être vivant.

### État de la technique

WO2011/075104 décrit un dispositif permettant de délivrer une solution médicamenteuse à un patient. Ce dispositif comprend un corps ayant un réservoir pour contenir ladite solution médicamenteuse, voir par exemple la revendication 1. Le dispositif est de préférence fourni à un patient dans une configuration pré-remplie. Pour remplir le réservoir, il est possible d'envisager un dispositif comprenant un port d'injection tel que décrit au paragraphe [0063]. Le dispositif de ce document comprend un réservoir incorporé. Le dispositif de WO2011/075104 n'est pas apte à recevoir et presser une poche contenant un liquide à perfuser.

La perfusion est une technique d'intervention, bien connue dans le domaine médical, pour l'injection lente et continue d'un liquide, tel qu'un médicament ou un liquide biologique, dans un organisme. Une telle intervention se pratique couramment, notamment en médecine humaine. Elle est en fait destinée à compenser des pertes de liquides biologiques, par exemple de sang, subies par un organisme à la suite, entre autres, d'une intervention chirurgicale, d'un accident de la route ou de toute circonstance ayant entraîné de telles pertes. C'est notamment aussi le cas dans des situations de conflit armé. Il est par ailleurs fréquent que l'organisme qui reçoit la perfusion se trouve en état de choc.

Par principe, la perfusion consiste en un écoulement d'un liquide approprié, contenu dans une poche souple, depuis cette poche jusqu'à l'organisme récepteur, via un circuit défini appelé aussi ligne de perfusion. Cet écoulement doit se faire avec un débit aussi constant que possible, adapté aux besoins de l'organisme qui le reçoit.

Selon la pratique habituelle, la poche de liquide est maintenue à une certaine hauteur au-dessus de l'organisme à perfuser, de façon à assurer un écoulement du liquide par gravité. Cette méthode s'applique assez facilement en milieu hospitalier ou analogue, où la poche peut être suspendue à une hauteur constante à une potence et accompagner le patient dans ses déplacements éventuels. En extérieur, par exemple sur les lieux d'un accident de la route ou d'une catastrophe naturelle, le maintien de la poche à la hauteur désirée mobilise un membre du personnel d'intervention, qui n'est dès lors plus disponible pour des missions de soins proprement dites. De plus, il n'est guère possible d'assurer un débit constant de liquide dans ces conditions.

Par ailleurs, particulièrement avec des poches de grandes dimensions, cette méthode ne permet pas toujours de garantir un débit suffisamment constant, même si la poche est maintenue à une hauteur constante, puisque la pression du liquide - et dès lors son débit - diminue au fur et à mesure que la poche se vide. Enfin, il existe un risque non négligeable que le patient, par un geste incontrôlé, arrache la ligne de perfusion qui le relie à la poche suspendue.

WO94/08645 divulgue un appareil autonome de perfusion permettant d'adresser ces problèmes. Cet appareil autonome de perfusion comporte des moyens pour recevoir et mettre une poche de perfusion sous pression entre deux plateaux de compression essentiellement parallèles. Lorsque cet appareil se trouve dans sa position normale de repos, par exemple rangé horizontalement dans une armoire, les plateaux de compression sont essentiellement horizontaux. Pendant la perfusion, le plateau supérieur reste fixe et le plateau inférieur, portant la poche, peut se déplacer verticalement vers le plateau supérieur sous l'action d'un mécanisme élastique. Pour charger une poche de perfusion dans l'appareil, il faut soulever le plateau supérieur, qui constitue un couvercle rabattable, afin d'avoir accès au plateau inférieur pour y déposer la poche de perfusion. La fermeture de ce couvercle rabattable provoque l'armement du mécanisme élastique et la mise sous pression de la poche de perfusion. On pourrait donc qualifier la poche d'externe à l'appareil en ce sens qu'il est possible de la retirer de l'appareil, par exemple, lorsqu'elle est vide.

L'appareil autonome de perfusion de WO94/08645 présente certains inconvénients. Il ne permet pas d'avoir un écoulement du liquide de la poche de perfusion avec un débit constant pendant toute la perfusion. D'autre part, il est difficile de vider complètement la poche de perfusion avec ce type d'appareil.

Le document DE 10058121 divulgue un appareil autonome de perfusion qui est conçu pour recevoir et mettre une poche de perfusion sous pression entre un plateau et une membrane élastique (8) partiellement retenue par une coque (1) percée d'une grande ouverture (7). Cet appareil nécessite de placer et de tendre ladite membrane dans l'appareil avant d'y placer la poche de perfusion, ce qui est relativement compliqué et couteux à l'usage car la membrane est une pièce qui doit être remplacée périodiquement (voir le paragraphe [0035] de ce document). Par ailleurs, la grande ouverture dans la coque implique que la membrane est exposée à des chocs extérieurs, ce qui rend le dispositif fragile. Enfin, la pression exercée par le plateau supérieur (14) sur la membrane, via la poche de perfusion, peut entrainer une hernie de la membrane au niveau de l'ouverture, ce qui la fragilise ou pourrait même la détruire.

### Résumé de l'invention

Un des buts de la présente invention est de fournir un appareil autonome apte à recevoir une poche externe contenant un fluide et apte à la presser pour véhiculer ledit fluide de ladite poche vers un être vivant, qui présente les avantages suivants :
- écoulement dudit fluide à partir de la poche le contenant avec un débit plus constant, et
- possibilité de vider plus complètement ladite poche.

A cette fin, l'appareil autonome selon l'invention comprend :
- un premier plateau ayant une première surface,
- un deuxième plateau ayant une deuxième surface, ladite deuxième surface du deuxième plateau étant convexe d'un point de vue situé entre lesdites première et deuxième surfaces desdits premier et deuxième plateaux,
ledit appareil autonome étant configuré :
- de sorte que ladite première surface du premier plateau et ladite deuxième surface du deuxième plateau sont aptes à se faire face, et
- de sorte qu'il est possible d'insérer ladite poche externe contenant le fluide, entre lesdites première et deuxième surfaces desdits premier et deuxième plateaux ;
ledit appareil autonome comprenant en outre un mécanisme élastique :
- pour rapprocher les première et deuxième surfaces des premier et deuxième plateaux, et
- pour mettre ladite poche sous pression entre la première surface du premier plateau et la deuxième surface du deuxième plateau.

L'appareil autonome de l'invention est caractérisé en ce que ladite deuxième surface (220) du deuxième plateau (22) est courbe en tout point de ladite deuxième surface (220) du deuxième plateau (22) qui fait face à la première surface (210) du premier plateau (21).

Ladite poche pourrait être qualifiée d'externe en ce sens qu'il est possible de l'enlever ou de la retirer de l'appareil autonome, par exemple lorsqu'elle est vide. En particulier, il est possible d'insérer et de presser différentes poches, l'une après l'autre typiquement, avec l'appareil autonome de l'invention.

Grâce à la configuration de l'appareil autonome et à son mécanisme élastique, il est possible de contenir ou maintenir une poche entre les première et deuxième surfaces des premier et deuxième plateaux. Il est possible d'insérer ladite poche entre les première et deuxième surfaces des premier et deuxième plateaux par exemple en écartant les premier et deuxième plateaux l'un de l'autre. Cela est par exemple possible en relevant le premier plateau lorsque ce dernier est un couvercle rabattable autour d'une charnière. De préférence, cela a pour effet de désarmer le mécanisme élastique.

Le terme convexe est connu de l'homme du métier. Ainsi, d'un point de vue situé entre les première et deuxième surfaces des premier et deuxième plateaux, la deuxième surface du deuxième plateau présente une surface bombée, c'est-à-dire une surface qui est arrondie. L'adjectif convexe s'oppose à l'adjectif concave, ce dernier désignant une surface dont la forme est en creux.

La forme convexe et courbede la deuxième surface du deuxième plateau d'un point de vue situé entre les première et deuxième surfaces des premier et deuxième plateaux permet d'avoir une meilleure coaptation des première et deuxième surfaces des premier et deuxième plateaux lorsqu'une poche est mise sous pression entre ces deux surfaces, par rapport à l'appareil de WO94/08645. La coaptation désigne l'action ou la propriété pour deux surfaces de se faire face, de s'ajuster, de coïncider. Au final, une poche mise sous pression entre les première et deuxième surfaces des premier et deuxième plateaux s'étalera (ou s'étendra) mieux avec l'appareil autonome de l'invention. Cela permet d'une part d'avoir une répartition de pression plus homogène sur la poche. La répartition de pression sur la poche est aussi plus stable au fur et à mesure que la poche se vide (en d'autres termes, la pression appliquée sur la poche est plus constante ou varie moins au fur et à mesure que ladite poche se vide). Ainsi, on peut obtenir un débit d'écoulement plus constant avec l'appareil autonome selon l'invention. D'autre part, la poche s'étalant (ou s'étirant) mieux entre les première et deuxième surfaces des premier et deuxième plateaux, le risque de formation de plis dans la poche au fur et à mesure qu'elle se vide est réduit. Or, des quantités de fluide à véhiculer peuvent être piégées dans de tels plis. Ainsi, l'appareil autonome de l'invention permet de vider plus complètement une poche vu que le risque de formation de plis dans la poche est réduit.

De préférence, ledit être vivant est un mammifère. De manière encore préférée, ledit être vivant est un être humain.

De préférence, ledit fluide est un liquide.

De préférence, l'appareil autonome est un appareil autonome de perfusion. Dans ce cas, le fluide à véhiculer vers un être vivant est généralement un liquide à perfuser et la poche est généralement une poche de perfusion.

De préférence, l'appareil autonome de l'invention est utilisé pour permettre une nutrition entérale. Ce terme est connu de l'homme du métier. La nutrition entérale est une méthode de substitution de l'alimentation orale permettant d'apporter tous les nutriments nécessaires à un organisme afin d'atteindre et de respecter un état nutritionnel correspondant aux besoins et aux caractéristiques de l'être vivant à alimenter. Pour une telle nutrition entérale, une sonde est généralement introduite dans le tube digestif par voie nasale ou par l'intermédiaire d'une stomie digestive. L'appareil autonome de l'invention permet de véhiculer les nutriments présents dans la poche vers l'être vivant à alimenter par l'intermédiaire d'une telle sonde.

De préférence, le deuxième plateau est suffisamment rigide pour ne pas se déformer substantiellement lorsqu'une poche est mise sous pression entre les première et deuxième surfaces des premier et deuxième plateaux.

Ladite première surface du premier plateau est concave et courbe en tout point de la première surface du premier plateau qui fait face à la deuxième surface du deuxième plateau en l'absence de poche et d'un point de vue situé entre lesdites première et deuxième surfaces des premier et deuxième plateaux.

Ce mode de réalisation préféré se rapporte préférentiellement au cas préféré où le premier plateau est essentiellement rigide, c'est-à-dire au cas préféré où ce premier plateau subit peu (ou pas du tout) de déformation suite à la mise sous pression d'une poche par le mécanisme élastique entre la première surface du premier plateau et la deuxième surface du deuxième plateau. Dans ce mode de réalisation préféré, la coaptation entre la première surface du premier plateau et la deuxième surface du deuxième plateau est encore meilleure car la première surface du premier plateau est concave tandis que la deuxième surface du deuxième plateau est convexe d'un point de vue situé entre ces deux surfaces. Ainsi, le débit d'écoulement du fluide peut être rendu encore plus constant et il est possible de vider encore plus complètement la poche. De manière encore préférée, la première surface du premier plateau et la deuxième surface du deuxième plateau sont essentiellement parallèles (deux surfaces sont parallèles si la normale à l'une est normale à l'autre), notamment lorsqu'une poche est mise sous pression entre ces deux surfaces.

Le premier plateau est apte à se déformer lorsqu'une poche est mise sous pression par le mécanisme élastique entre la première surface du premier plateau et la deuxième surface du deuxième plateau de sorte que ladite première surface du premier plateau est concave et courbe en tout point de la première surface du premier plateau qui fait face à la deuxième surface du deuxième plateau en présence d'une poche mise sous pression entre la première surface du premier plateau et la deuxième surface du deuxième plateau et d'un point de vue situé entre lesdites première et deuxième surfaces des premier et deuxième plateaux.

Le premier plateau est apte à se déformer lorsqu'une poche est mise sous pression pour adopter une forme concave et courbe en tout point de la première surface du premier plateau qui fait face à la deuxième surface du deuxième plateau d'un point de vue situé entre les deux surfaces pressant la poche. Quand une poche est mise sous pression avec l'appareil autonome de perfusion de WO94/08645, le couvercle rabattable (ou premier plateau) se déforme et prend une forme de type 'panse de vache'. Cette déformation résulte de la pression exercée par la poche de perfusion sur le couvercle rabattable et est d'autant plus marquée lorsque le couvercle (ou premier plateau) est fait d'un matériau plastique léger et transparent comme cela est préféré pour ce type d'application. Il est en effet préféré d'avoir un appareil autonome de perfusion qui ne soit pas trop lourd et d'avoir un couvercle (ou premier plateau) transparent pour surveiller le degré de remplissage de la poche de perfusion. Or, le plateau mobile (ou deuxième plateau) de l'appareil de WO94/08645 est plan. Au final, la coaptation entre la surface inférieure du couvercle rabattable (ou premier plateau) et la surface supérieure du plateau mobile (ou deuxième plateau) est mauvaise. Elle est en tout cas moins bonne que celle obtenue avec le mode de réalisation préféré de l'invention décrit dans le présent paragraphe. La deuxième surface du deuxième plateau étant convexe et la première surface du premier plateau étant concave lorsqu'une poche est mise sous pression, la coaptation entre ces deux surfaces est meilleure. Au final, le débit d'écoulement du fluide à véhiculer est plus constant et le risque de formation de plis pouvant piéger du fluide est réduit. De manière encore préférée, la première surface du premier plateau et la deuxième surface du deuxième plateau sont essentiellement parallèles lorsqu'une poche est mise sous pression par le mécanisme élastique entre la première surface du premier plateau et la deuxième surface du deuxième plateau (deux surfaces sont parallèles si la normale à l'une est normale à l'autre).

La première surface du premier plateau a une forme convexe en l'absence de poche et d'un point de vue situé entre lesdites première et deuxième surfaces des premier et deuxième plateaux.

De préférence, des intersections de plans de coupe avec la deuxième surface du deuxième plateau définissent des courbes du deuxième degré dans des repères orthogonaux situés dans ces plans de coupe.
De préférence, des intersections de plans de coupe avec la première surface du premier plateau définissent des courbes du deuxième degré dans des repères orthogonaux compris dans ces plans de coupe.

De préférence, la convexité de la deuxième surface du deuxième plateau (d'un point de vue situé entre les première et deuxième surfaces des premier et deuxième plateaux) est telle que cette deuxième surface du deuxième plateau a une flèche comprise entre 2 et 3 mm.

De préférence, la convexité de la deuxième surface du deuxième plateau (d'un point de vue situé entre les première et deuxième surfaces des premier et deuxième plateaux) est telle que cette deuxième surface du deuxième plateau a une flèche comprise entre 1 et 2 mm, et de manière encore préférée égale à 1.5 mm.

De préférence, le premier plateau est un couvercle. De manière encore préférée, le premier plateau est un couvercle rabattable autour d'une charnière.

De préférence, l'appareil autonome comprend en outre un boîtier comprenant un fond.

De préférence, le deuxième plateau est mobile par rapport au fond du boîtier et ledit mécanisme élastique est situé entre le fond du boîtier et ledit deuxième plateau.

De préférence, le premier plateau est constitué d'un matériau comprenant une matière plastique.

De préférence, le premier plateau est constitué d'un matériau comprenant une matière plastique chargée en fibres de verre.

De préférence, le deuxième plateau est constitué d'un matériau comprenant une matière plastique.

De préférence, le deuxième plateau est constitué d'un matériau comprenant une matière plastique chargée en fibres de verre.

Le poids de l'appareil autonome peut être réduit quand le premier plateau et/ou le deuxième plateau est (sont) constitué(s) d'un matériau comprenant une matière plastique. En utilisant une matière plastique chargée en fibres de verre pour le premier et/ou le deuxième plateau(x), il est possible d'avoir un appareil autonome solide et léger à la fois.

De préférence, la deuxième surface du deuxième plateau comprend au moins un ergot pour fixer une poche apte à contenir le fluide à véhiculer vers un être vivant. La présence d'un tel ergot permet de stabiliser une poche pressée entre les deux plateaux.

De préférence, le premier plateau comprend au moins une portion transparente. Avec ce mode de réalisation préféré, il est possible d'observer le degré de remplissage d'une poche insérée dans l'appareil autonome, et cela facilement.

De préférence, le mécanisme élastique est couplé directement au deuxième plateau.

De préférence, l'appareil autonome comprend en outre des moyens de surveillance pour contrôler le degré de vidange d'une poche.

De préférence, une projection de la deuxième surface du deuxième plateau sur un plan a essentiellement la forme d'un rectangle.

De préférence, la première surface du premier plateau est une surface inférieure du premier plateau.

De préférence, la deuxième surface du deuxième plateau est une surface supérieure du deuxième plateau.

### Brève description des figures

Ces aspects ainsi que d'autres aspects de l'invention seront clarifiés dans la description détaillée de modes de réalisation préférés de l'invention, référence étant faite aux dessins des figures, dans lesquelles :
- la Fig.1: montre schématiquement une coupe d'un appareil autonome selon un premier mode de réalisation préféré en l'absence et avec une poche;
- la Fig.2: montre schématiquement une coupe d'un appareil autonome selon un autre mode de réalisation préféré en l'absence et avec une poche;
- la Fig.3: illustre la détermination d'une flèche caractérisant la déformation convexe de la deuxième surface du deuxième plateau ;
- la Fig.4: montre une vue en perspective de la deuxième surface du deuxième plateau pour un mode de réalisation préféré de l'invention ainsi qu'une coupe d'une telle deuxième surface selon un plan vertical ;
- la Fig. 5: montre une vue du dessus de la deuxième surface du deuxième plateau selon un autre mode de réalisation préféré ;
- la Fig. 6: montre une coupe d'un autre mode de réalisation préféré de l'appareil de l'invention en l'absence et avec une poche;
- la Fig. 7: montre une coupe d'un autre mode de réalisation préféré de l'appareil de l'invention en l'absence et avec une poche.

Les dessins des figures ne sont pas à l'échelle. Généralement, des éléments semblables sont dénotés par des références semblables dans les figures. La présence de numéros de référence aux dessins ne peut être considérée comme limitative, y compris lorsque ces numéros sont indiqués dans les revendications.

### Description détaillée de modes de réalisation particuliers

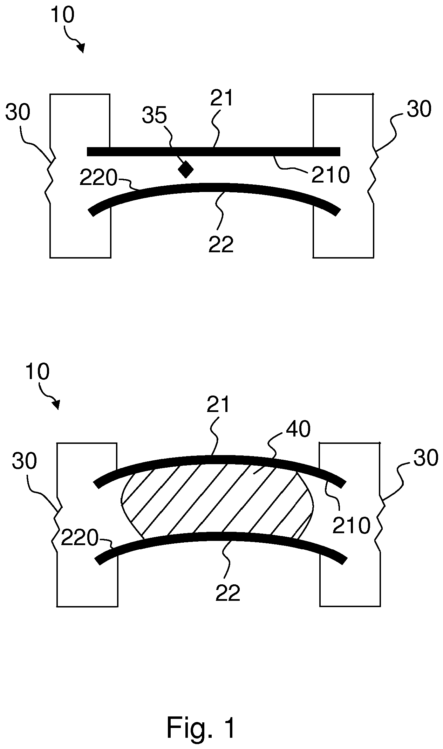

La figure 1 montre schématiquement l'appareil autonome 10 selon un premier mode de réalisation préféré. La partie haute de la figure 1 représente l'appareil autonome 10 en l'absence de poche 40. L'appareil autonome 10 comprend un premier plateau 21 ayant une première surface 210. Il comprend aussi un deuxième plateau 22 ayant une deuxième surface 220. Un mécanisme élastique 30 permet de rapprocher la première surface 210 du premier plateau 21 vers la deuxième surface 220 du deuxième plateau 22. Comme on peut le voir à la partie basse de figure 1, ce mécanisme élastique 30 permet aussi de mettre une poche 40 sous pression entre la première surface 210 du premier plateau 21 et la deuxième surface 220 du deuxième plateau 22. Cette poche 40 permet de contenir un fluide qui doit être véhiculé vers un être vivant. Comme on peut le voir à la figure 1, le premier plateau 21 est préférentiellement apte à se déformer lorsqu'une poche 40 est mise sous pression par le mécanisme élastique 30 entre la première surface 210 du premier plateau 21 et la deuxième surface 220 du deuxième plateau 22. L'appareil autonome 10 est configuré de sorte que la première surface 210 du premier plateau 21 et la deuxième surface 220 du deuxième plateau 22 peuvent se faire face. La deuxième surface 220 du deuxième plateau 22 est convexe d'un point de vue 35 situé entre les première 210 et deuxième 220 surfaces des premier 21 et deuxième 22 plateaux. Ainsi, du point de vue 35, la deuxième surface 220 du deuxième plateau 22 a une forme de bosse, elle est bombée ; elle n'est pas concave, elle ne présente pas un creux. Une fois qu'une poche 40 est mise sous pression entre les deux surfaces 210 et 220, le premier plateau 21 se déforme dans le mode de réalisation préféré illustré à la figure 1 à cause de la pression exercée par la poche 40 et cette dernière est pressée entre deux surfaces 210 et 220 qui présentent une meilleure coaptation par rapport à l'appareil décrit dans WO94/08645. Cela est possible grâce à la forme convexe de la deuxième surface 220 du deuxième plateau 22 du point de vue 35.

Dans le mode de réalisation préféré qui est illustré à la figure 1, la première surface 210 du premier plateau 21 est essentiellement plane en l'absence de poche 40. Dans un autre mode de réalisation préféré (non représenté), la première surface 210 du premier plateau 21 est convexe en l'absence de poche 40 et d'un point de vue 35 situé entre les deux surfaces 210 et 220. Dans ce même mode de réalisation préféré, le premier plateau 21 est apte à se déformer lorsqu'une poche 40 est mise sous pression par le mécanisme élastique 30 entre la première surface 210 du premier plateau 21 et la deuxième surface 220 du deuxième plateau 22 de sorte que la première surface 210 du premier plateau 21 est concave lorsqu'une poche 40 est mise sous pression entre les deux surfaces 210 et 220. Ainsi, lorsqu'une poche 40 est mise sous pression entre les deux surfaces 210 et 220, il y a également une bonne coaptation entre ces deux surfaces 210 et 220.

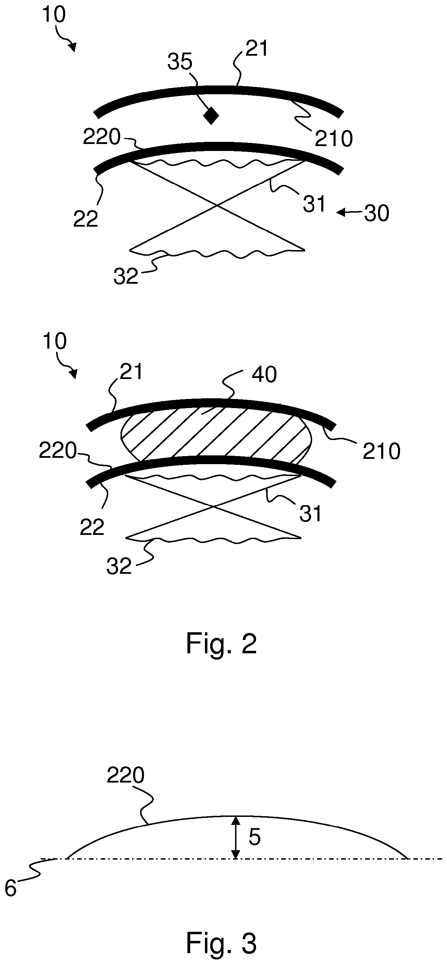

La figure 2 montre un autre mode de réalisation préféré. Comme on peut le voir sur cette figure, la première surface 210 du premier plateau 21 est de préférence concave en l'absence de poche 40 et d'un point de vue 35 situé entre les première 210 et deuxième 220 surfaces des premier 21 et deuxième plateau 22. La deuxième surface 220 du deuxième plateau 22 est convexe du même point de vue 35. Ainsi, au final, la répartition de pression sur une poche 40 mise sous pression entre les deux surfaces 210 et 220 est plus homogène et il est possible de vider la poche 40 en limitant le risque de formation de plis dans lesquels du fluide à véhiculer peut être retenu. Le mécanisme élastique 30 montré à la figure 1 pourrait être utilisé avec le mode de réalisation préféré montré à la figure 2 et inversement. Ainsi, le mécanisme élastique 30 de la figure 2 qui comprend deux leviers 31 croisés réunis par des ressorts 32 en traction pourrait être utilisé avec le mode de réalisation préféré qui est montré à la figure 1. Dans le mode de réalisation montré à la figure 2, un mécanisme (non représenté) permet de maintenir le premier plateau 21 dans une certaine position de manière à ce qu'il exerce une pression sur la poche 40 qui elle-même imprime une pression sur la deuxième surface 220 du deuxième plateau 22 permettant la mise sous tension des ressorts 32 qui ont tendance à repousser le deuxième plateau 22 vers le premier plateau 21. Il peut par exemple s'agir d'un mécanisme d'armement qui permet de bloquer le premier plateau 21 dans une certaine position.

De préférence, la convexité de la deuxième surface 220 du deuxième plateau 22 est telle que cette deuxième surface 220 a une flèche 5 comprise entre 2 et 3 mm pour tous les modes de réalisation préférés de l'invention. Cependant, cette flèche 5 peut être plus élevée. Ainsi par exemple, cette flèche 5 peut valoir 3.5, 4, 4.5 ou 5 mm. La flèche 5 est définie comme la valeur maximale de la déformation de la deuxième surface 220 du deuxième plateau 22 par rapport à une surface plane 6. La figure 3 montre comment cette flèche 5 est déterminée. De préférence, pour le mode de réalisation préféré montré à la figure 2, la concavité de la première surface 210 du premier plateau 21 est telle que cette première surface 210 a une flèche comprise entre 2 et 3 mm. Cependant, cette flèche de la première surface 210 peut être plus élevée. Ainsi par exemple, cette flèche peut valoir 3.5, 4, 4.5 ou 5 mm.

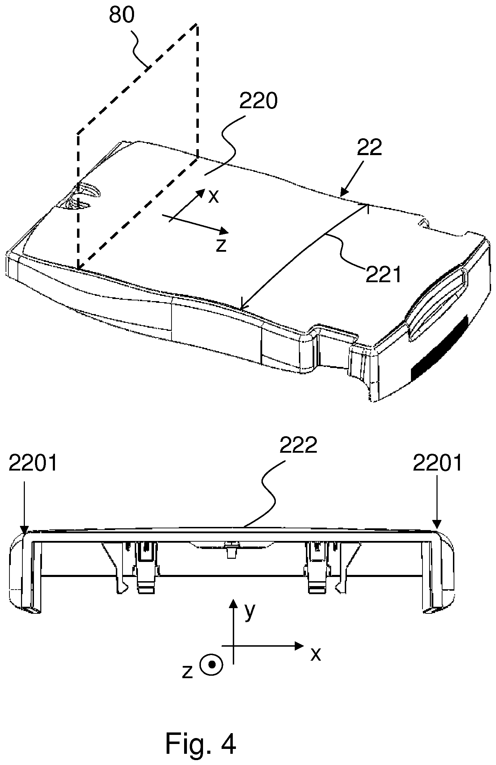

Dans une autre version préférée, la deuxième surface 220 du deuxième plateau 22 qui est convexe d'un point de vue 35 situé entre les deux surfaces 210 et 220 a une forme telle que des intersections de plans de coupe 80 avec cette deuxième surface 220 définissent des courbes du deuxième degré dans des repères orthogonaux situés dans ces plans de coupe 80. Cette version préférée est illustrée à la figure 4. Le haut de la figure 4 montre une vue en perspective de la deuxième surface 220 du deuxième plateau 22 pour ce mode de réalisation préféré de l'invention ainsi qu'un plan de coupe 80 vertical. Le bas de la figure 4 montre l'intersection de ce plan de coupe 80 vertical avec la deuxième surface 220 du deuxième plateau 22. Cette intersection du plan de coupe 80 avec la deuxième surface 220 du deuxième plateau 22 définit une courbe 222. De préférence, cette courbe 222 est une courbe du deuxième degré dans le repère orthogonal x-y montré au bas de la figure 4. Afin de quantifier la déformation convexe de la deuxième surface 220, nous choisissons une origine de l'axe x qui est située à la moitié de la largeur moyenne 221 de la deuxième surface 220 du deuxième plateau 22. Ainsi, la distance curviligne entre x=0 et chacune des deux extrémités 2201 de la courbe 222 montrées au bas de la figure 4 est la même (en supposant que les deux extrémités 2201 sont séparées par la largeur moyenne 221). De préférence, si ces deux extrémités 2201 de la courbe 222 sont situées à y=0, le point de la courbe 222 de coordonnée x=0 a une coordonnée y comprise entre 0.5 et 3 mm (cela revient à dire que la flèche 5 est comprise entre 0.5 et 3 mm). De manière encore préférée, si ces deux extrémités 2201 de la courbe 222 sont situées à y=0, le point de la courbe 222 en x=0 a une coordonnée y comprise entre 1 et 2.5 mm (cela revient à dire que la flèche 5 est comprise entre 1 et 2.5 mm). De préférence, la déformation convexe de la deuxième surface 220 du deuxième plateau 22 n'est pas constante (ou uniforme) le long de l'axe z, c'est-à-dire le long de la longueur du deuxième plateau.

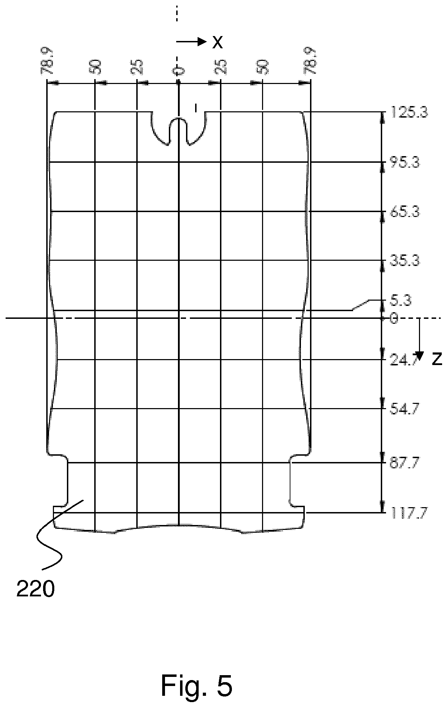

Dans une autre version préférée, la deuxième surface 220 du deuxième plateau 22 a une forme telle que des intersections de plans de coupe 80 avec cette deuxième surface 220 définissent des courbes du deuxième degré dans des repères orthogonaux x-y situés dans ces plans de coupe 80, ces courbes du deuxième degré ayant les équations suivantes aux différentes coordonnées z montrées à la figure 5 (un exemple de plan de coupe 80 est montré en haut de la figure 4). La figure 5 est une vue du dessus de la deuxième surface 220 du deuxième plateau 22 où l'on peut visualiser l'origine des axes x et z (les coordonnées le long de ces axes sont exprimées en mm). L'axe y est quant à lui perpendiculaire au plan de la figure 5 (et donc perpendiculaire aux axes x et z).

Equations du deuxième degré dans le repère x-y définissant les intersections de la deuxième surface 220 avec des plans de coupe 80 situés à différentes coordonnées z pour le mode de réalisation préféré illustré à la figure 5 :
- en z=117.7 mm : y=-0.0003 x² - 5*10⁻⁵ x + 2.5407 ;
- en z=87.7 mm : y=-0.0003 x² - 6*10⁻⁵ x + 2.4888 ;
- en z=54.7 mm : y=-0.0003 x² - 6*10⁻⁵ x + 2.4555 ;
- en z=24.7 mm : y=-0.0002 x² - 4*10⁻⁵ x + 2.417 ;
- en z=-5.3 mm : y=-0.0002 x² - 4*10⁻⁵ x + 2.3152 ;
- en z=-35.3 mm : y=-0.0002 x² - 4*10⁻⁵x + 2.1111 ;
- en z=-65.3 mm : y=-0.0001 x² - 4*10⁻⁵ x + 1.7707 ;
- en z=-95.3 mm : y=- 7*10-⁵ x² - 10⁻⁵ x + 1.2648 ;
- en z=-125.3 mm : y=- 2*10⁻⁵ x² - 7*10⁻⁶ x + 0.5742.

Dans ces différentes équations, les coordonnées x et y sont exprimées en mm. En calculant la différence des coordonnées y pour x=0 et x=78.9 mm, on peut déduire des estimations des différentes flèches 5 aux différentes coordonnées z. Ainsi, en z= 117.7 mm, on déduit que la flèche 5 est d'environ 0,669 mm (en remplaçant dans la première équation x par 78.9 mm).

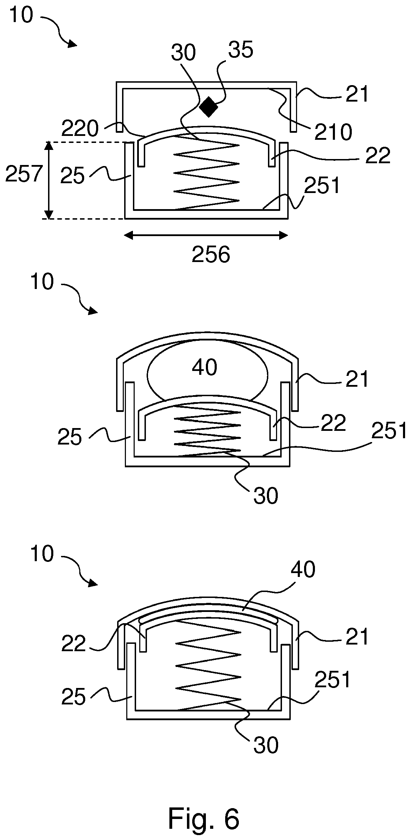

La figure 6 montre une coupe d'un autre mode de réalisation préféré de l'appareil autonome 10 de l'invention en l'absence et avec une poche 40. Dans ce mode de réalisation préféré, le premier plateau 21 est apte à se déformer lorsqu'une poche 40 est mise sous pression par le mécanisme élastique 30 entre la première surface 210 du premier plateau 21 et la deuxième surface 220 du deuxième plateau 22 de sorte que ladite première surface 210 du premier plateau 21 est concave en présence d'une poche 40 mise sous pression entre les deux surfaces 210 et 220 d'un point de vue 35 située entre ces deux surfaces 210 et 220. Dans ce mode de réalisation préféré, l'appareil autonome 10 comprend en outre un boîtier 25 qui comprend un fond 251. Le premier plateau 21 est un couvercle rabattable couplé en boîtier 25. Le deuxième plateau 22 est mobile par rapport au fond 251 du boîtier 25 et le mécanisme élastique 30 est situé entre le fond 251 du boîtier 25 et le deuxième plateau 22. L'illustration haute de la figure 6 montre l'appareil autonome 10 sans poche 40 tandis les illustrations médiane et basse de la figure 6 montrent l'appareil autonome 10 quand une poche 40 est mise sous pression entre la première surface 210 du premier plateau 21 et la deuxième surface 220 du deuxième plateau 22. L'illustration médiane de la figure 6 correspond au cas où la poche 40 est essentiellement remplie, tandis que l'illustration basse de la figure 6 montre la configuration où la poche 40 est essentiellement vide. Un système d'armement (non représenté) permet de maintenir le premier plateau 21 (couvercle rabattable) dans une position fixe par rapport au boîtier 25 lorsqu'il est refermé sur le dessus de ce dernier (voir les illustrations médiane et basse de la figure 6). Grâce à une telle position fixe du premier plateau 21, une poche 40 peut être mise sous pression entre les première 210 et deuxième 220 surfaces des premier 21 et deuxième 22 plateaux par la mise sous tension du mécanisme élastique 30. De préférence, l'appareil autonome 10 comporte des moyens de verrouillage du deuxième plateau 22 pour le maintenir dans une position d'armement permettant de poser la poche 40. La position d'armement du deuxième plateau 22 mobile est la position dans laquelle il se trouve lorsque le mécanisme élastique 30 exerce une force de rappel maximale pour rapprocher la deuxième surface 220 du deuxième plateau 22 vers la première surface 210 du premier plateau 21. Pour le mode de réalisation préféré de la figure 6 positionné comme cela est illustré à la même figure 6, cette position d'armement correspond à une position basse du deuxième plateau 22. Dans cette variante préférée, l'appareil autonome 10 comporte aussi des moyens pour débloquer ledit deuxième plateau 22 de sa position d'armement en vue de permettre son mouvement vers sa position de repos. La position de repos du deuxième plateau 22 mobile est la position dans laquelle il se trouve lorsque le mécanisme élastique 30 exerce une force de rappel minimale pour rapprocher la deuxième surface 220 du deuxième plateau 22 vers la première surface 210 du premier plateau 21. La position de repos correspond généralement à la position pour laquelle une poche 40 placée entre les première 210 et deuxième 220 surfaces est essentiellement vide. Lorsqu'un mécanisme élastique 30 tel que celui illustré à la figure 2 est utilisé, la position d'armement correspond à la situation où les ressorts 32 sont tendus au maximum, tandis que la position de repos correspond à la situation où les ressorts 32 sont tendus au minium.

De préférence, le boîtier 25 a une longueur de 150 à 300 mm (mesurée perpendiculairement au plan de la figure 6). De préférence, des coupes du boîtier 25 perpendiculaires à cette longueur définissent des sections en forme de U comme cela est illustré à la figure 6. De préférence, la largeur 256 de cette forme de U est comprise entre 100 et 200 mm. De préférence, la hauteur 257 de cette forme de U est comprise entre 40 et 80 mm. Ces dimensions préférées permettent à l'appareil 10 de recevoir des poches de perfusion 40 utilisées couramment.

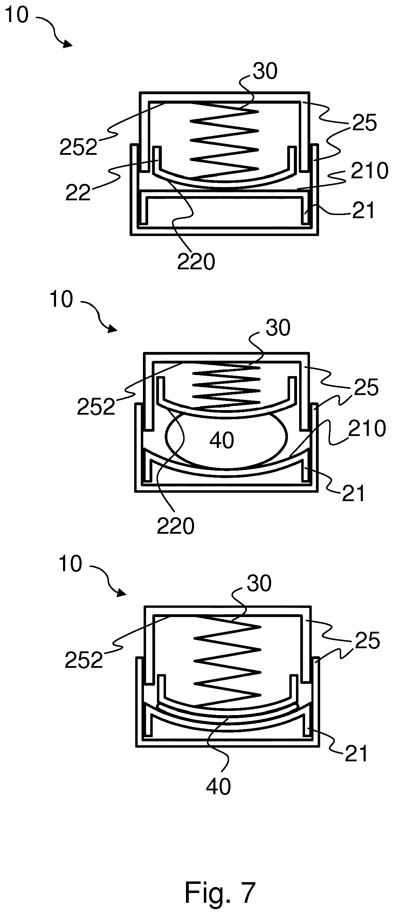

La figure 7 montre un autre mode de réalisation préféré de l'appareil autonome 10. Comme on peut le voir à cette figure, le mécanisme élastique 30 est situé entre la paroi supérieure 252 du boîtier 25 et le deuxième plateau 22. Une poche 40 peut être déposée sur la première surface 210 du premier plateau 21. Dans ce mode de réalisation préféré, le premier plateau 21 est apte à coulisser à la manière d'un tiroir. Cela permet d'insérer la poche 40 dans l'appareil autonome 10. Dans ce mode de réalisation préféré qui illustré à la figure 7, le premier plateau 21 subit une déformation concave quand une poche 40 est mise sous pression entre les première 210 et deuxième 220 surfaces.

De préférence, la deuxième surface 220 du deuxième plateau 22 comprend au moins un ergot pour fixer une poche 40. De manière encore préférée, la deuxième surface 220 du deuxième plateau 22 comprend deux tels ergots.

De préférence, le premier plateau 21 est constitué essentiellement d'une matière plastique. Des exemples de tels matériaux plastiques sont : polyéthylène, polyamides. De manière encore préférée, le premier plateau 21 est constitué essentiellement d'une matière plastique chargée. De manière encore plus préférée, le premier plateau 21 est constitué essentiellement d'une matière plastique chargée en fibres de verre. Dans une autre version préférée, le premier plateau 21 est constitué essentiellement d'une matière plastique transparente. Cette matière plastique transparente peut comprendre une matière plastique transparente chargée en fibres de verre.

De préférence, le deuxième plateau 22 est constitué essentiellement d'une matière plastique. De manière encore préférée, le deuxième plateau 22 est constitué essentiellement d'une matière plastique chargée. De manière encore plus préférée, le deuxième plateau 22 est constitué essentiellement d'une matière plastique chargée en fibres de verre.

De préférence, le premier plateau 21 comprend au moins une portion transparente. Cela permet de visualiser le taux de remplissage de la poche 40.

De préférence, l'appareil autonome 10 comprend des moyens de surveillance pour contrôler le degré de vidange d'une poche 40. Des exemples de tels moyens de surveillance pouvant être utilisés seuls ou en combinaison sont :
- un système composé d'une roue codeuse et d'un capteur optoélectronique détectant la variation de hauteur d'un des premier 21 ou deuxième 22 plateaux ;
- un système de signalisation sonore et/ou visuelle de la fin de la perfusion;
- un système de signalisation sonore et/ou visuelle de tout défaut survenant pendant la perfusion : perte de pression, bouchage de la ligne de perfusion, et analogues ;
- débitmètre, de préférence massique ;
- moyens d'affichage du débit ;
- système d'alarme en cas de variation du débit au-delà de limites prédéterminées.

L'appareil autonome 10 de l'invention n'a pas besoin d'être dans une position particulière pour fonctionner. Grâce à la mise sous pression d'une poche 40 entre les première 210 et deuxième 220 surfaces des premier 21 et deuxième 22 plateaux, l'appareil autonome 10 peut fonctionner dans n'importe quelle position. De préférence, le mécanisme élastique 30 comprend un mécanisme taré et auto-lubrifié de biellettes articulées, des éléments de ressorts tarés de façon à augmenter la force de pression tendant à rapprocher les première 210 et deuxième 220 surfaces des premier 21 et deuxième 22 plateaux.

La présente invention a été décrite en relation avec des modes de réalisations spécifiques, qui ont une valeur purement illustrative et ne doivent pas être considérés comme limitatifs. D'une manière générale, la présente invention n'est pas limitée aux exemples illustrés et/ou décrits ci-dessus. L'usage des verbes « comprendre », « inclure », « comporter », ou toute autre variante, ainsi que leurs conjugaisons, ne peut en aucune façon exclure la présence d'éléments autres que ceux mentionnés. L'usage de l'article indéfini « un », « une », ou de l'article défini « le », « la » ou « l' », pour introduire un élément n'exclut pas la présence d'une pluralité de ces éléments. Les numéros de référence dans les revendications ne limitent pas leur portée.

En résumé, l'invention peut également être décrite comme suit. Appareil autonome 10 comprenant un premier plateau 21 ayant une première surface 210, un deuxième plateau 22 ayant une deuxième surface 220, un mécanisme élastique 30 pour rapprocher les première 210 et deuxième 220 surfaces et pour mettre une poche 40 sous pression entre elles, l'appareil autonome 10 étant configuré de sorte que ladite première surface 210 et ladite deuxième surface 220 sont aptes à se faire face et étant caractérisé en ce que ladite deuxième surface 220 du deuxième plateau 22 est convexe et courbe en tout point de la deuxième surface (220) du deuxième plateau (22) qui fait face à la première surface (210) du premier plateau (21)d'un point de vue 35 situé entre lesdites première 210 et deuxième 220 surfaces desdits premier 21 et deuxième 22 plateaux.

## Revendications

1. Appareil autonome (10) apte à recevoir une poche externe (40) contenant un fluide et apte à la presser pour véhiculer ledit fluide de ladite poche (40) vers un être vivant, ledit appareil autonome (10) comprenant :
- un premier plateau (21) ayant une première surface (210),
- un deuxième plateau (22) ayant une deuxième surface (220),
ladite deuxième surface (220) du deuxième plateau (22) étant convexe d'un point de vue (35) situé entre lesdites première (210) et deuxième (220) surfaces desdits premier (21) et deuxième (22) plateaux et ladite deuxième surface (220) du deuxième plateau (22) est courbe en tout point de ladite deuxième surface (220) du deuxième plateau (22) qui fait face à la première surface (210) du premier plateau (21).
ledit appareil autonome (10) étant configuré:
- de sorte que ladite première surface (210) du premier plateau (21) et ladite deuxième surface (220) du deuxième plateau (22) sont aptes à se faire face, et
- de sorte qu'il est possible d'insérer ladite poche externe (40) contenant le fluide, entre lesdites première (210) et deuxième (220) surfaces desdits premier (21) et deuxième (22) plateaux ;
ledit appareil autonome (10) comprenant en outre un mécanisme élastique (30) :
- pour rapprocher les première (210) et deuxième (220) surfaces des premier (21) et deuxième (22) plateaux, et
- pour mettre ladite poche (40) sous pression entre la première surface (210) du premier plateau (21) et la deuxième surface (220) du deuxième plateau (22),
et où la première surface (210) est dépourvue d'ouverture en tout point qui fait face à la deuxième surface (220), et en ce que, soit la première surface est concave et courbe en tout point de la première surface (210) qui fait face à la deuxième surface (220) en l'absence de poche (40) et d'un point de vue (35) situé entre lesdites première (210) et deuxième (220) surfaces, soit le premier plateau (21) est apte à se déformer lorsqu'une poche (40) est mise sous pression par le mécanisme élastique (30) entre la première surface (210) et la deuxième surface (220).

2. Appareil autonome (10) selon la revendication 1 **caractérisé en ce que** ladite première surface (210) du premier plateau (21) est concave et courbe en tout point de la première surface (210) du premier plateau (21) qui fait face à la deuxième surface (220) du deuxième plateau (22) en l'absence de poche (40) et d'un point de vue (35) situé entre lesdites première (210) et deuxième (220) surfaces des premier (21) et deuxième (22) plateaux.

3. Appareil autonome (10) selon la revendication 1 **caractérisé en ce que** le premier plateau (21) est apte à se déformer lorsqu'une poche (40) est mise sous pression par le mécanisme élastique (30) entre la première surface (210) du premier plateau (21) et la deuxième surface (220) du deuxième plateau (22) de sorte que ladite première surface (210) du premier plateau (21) est concave et courbe en tout point de la première surface (210) du premier plateau (21) qui fait face à la deuxième surface (220) du deuxième plateau (22) en présence d'une poche (40) mise sous pression entre la première surface (210) du premier plateau (21) et la deuxième surface (220) du deuxième plateau (22) et d'un point de vue (35) situé entre lesdites première (210) et deuxième (220) surfaces des premier (21) et deuxième (22) plateaux.

4. Appareil autonome (10) selon la revendication 3 **caractérisé en ce que** la première surface (210) du premier plateau (21) a une forme convexe en l'absence de poche (40) et d'un point de vue (35) situé entre lesdites première (210) et deuxième (220) surfaces des premier (21) et deuxième (22) plateaux.

5. Appareil autonome (10) selon l'une quelconque des revendications précédentes **caractérisé en ce que** des intersections de plans de coupe (80) avec la deuxième surface (220) du deuxième plateau (22) définissent des courbes du deuxième degré dans des repères orthogonaux situés dans ces plans de coupe (80).

6. Appareil autonome (10) selon l'une quelconque des revendications précédentes **caractérisé en ce que** des intersections de plans de coupe (80) avec la première surface (210) du premier plateau (21) définissent des courbes du deuxième degré dans des repères orthogonaux compris dans ces plans de coupe (80).

7. Appareil autonome (10) selon l'une quelconque des revendications précédentes **caractérisé en ce que** ladite convexité de la deuxième surface (220) du deuxième plateau (22) est telle que cette deuxième surface (220) du deuxième plateau (22) a une flèche comprise entre 2 et 3 mm.

8. Appareil autonome (10) selon l'une quelconque des revendications 1 à 6 **caractérisé en ce que** la convexité de la deuxième surface (220) du deuxième plateau (22) est telle que cette deuxième surface (220) du deuxième plateau (22) a une flèche comprise entre 1 et 2 mm, de préférence égale à 1.5 mm.

9. Appareil autonome (10) selon l'une quelconque des revendications précédentes **caractérisé en ce que** le premier plateau (21) est un couvercle.

10. Appareil autonome (10) selon l'une quelconque des revendications précédentes **caractérisé en ce qu'**il comprend en outre un boîtier (25) comprenant un fond (251).

11. Appareil autonome (10) selon la revendication précédente **caractérisé en ce que** le deuxième plateau (22) est mobile par rapport au fond (251) du boîtier (25) et **en ce que** ledit mécanisme élastique (30) est situé entre le fond (251) du boîtier (25) et ledit deuxième plateau (22).

12. Appareil autonome (10) selon l'une quelconque des revendications précédentes **caractérisé en ce que** le premier plateau (21) est constitué d'un matériau comprenant une matière plastique.

13. Appareil autonome (10) selon l'une quelconque des revendications précédentes **caractérisé en ce que** le premier plateau (21) est constitué d'un matériau comprenant une matière plastique chargée en fibres de verre.

14. Appareil autonome (10) selon l'une quelconque des revendications précédentes **caractérisé en ce que** le deuxième plateau (22) est constitué d'un matériau comprenant une matière plastique.

15. Appareil autonome (10) selon l'une quelconque des revendications précédentes **caractérisé en ce que** la deuxième surface (220) du deuxième plateau (22) comprend un ergot pour fixer ladite poche (40).

## Patentansprüche

1. Autonomes Gerät (10), das geeignet ist, um einen eine Flüssigkeit enthaltenden externen Beutel (40) aufzunehmen und das geeignet ist, um ihn zu drücken, um die genannte Flüssigkeit von dem genannten Beutel (40) zu einem Lebewesen zu befördern, wobei das genannte autonome Gerät (10) umfasst:
- eine erste Platte (21) mit einer ersten Fläche (210),
- eine zweite Platte (22) mit einer zweiten Fläche (220),
wobei die genannte zweite Fläche (220) der zweiten Platte (22) von einem Blickwinkel (35) aus, der zwischen der genannten ersten (210) und der genannten zweiten (220) Fläche der genannten ersten (21) und der genannten zweiten (22) Platte angeordnet ist, konvex ist und die genannte zweite Fläche (220) der zweiten Platte (22) an jedem Punkt der genannten zweiten Fläche (220) der genannten zweiten Platte (22) gekrümmt ist, die der ersten Fläche (210) der ersten Platte (21) gegenüberliegt,
wobei das genannte autonome Gerät (10) ausgestaltet ist:
- derart, dass die genannte erste Fläche (210) der ersten Platte (21) und die genannte zweite Fläche (220) der zweiten Platte (22) geeignet sind, einander gegenüber zu sein und
- derart, dass es möglich ist, den genannten, die Flüssigkeit enthaltenden externen Beutel (40) zwischen der genannten ersten (210) und der genannten zweiten (220) Fläche der genannten ersten (21) und der genannten zweiten (22) Platte einzufügen;
wobei das genannte autonome Gerät (10) darüber hinaus einen elastischen Mechanismus (30) umfasst:
- um die erste (210) und die zweite (220) Fläche der ersten (21) und der zweiten (22) Platte einander anzunähern und
- um den genannten Beutel (40) zwischen der ersten Fläche (210) der ersten Platte (21) und der zweiten Fläche (220) der zweiten Platte (22) mit Druck zu beaufschlagen,
und wobei die erste Fläche (210) an jedem Punkt, der der zweiten Fläche (220) gegenüberliegt, keine Öffnung aufweist und dass entweder die erste Fläche an jedem Punkt der ersten Flüche (210), die der zweiten Fläche (220) gegenüberliegt, bei fehlendem Beutel (40) und von einem Blickwinkel (35), der zwischen der genannten ersten (210) und der genannten zweiten (220) Fläche angeordnet ist, konkav und gekrümmt ist, oder die erste Platte (21) geeignet ist, sich zu verformen, wenn ein Beutel (40) durch den elastischen Mechanismus (30) zwischen der ersten Fläche (210) und der zweiten Fläche (220) mit Druck beaufschlagt ist.

2. Autonomes Gerät (10) gemäß Anspruch 1, **dadurch gekennzeichnet, dass** die genannte erste Fläche (210) der ersten Platte (21) an jedem Punkt der ersten Fläche (210) der ersten Platte (21) konkav und gekrümmt ist, die der zweiten Fläche (220) der zweiten Platte (22) bei fehlendem Beutel (40) und von einem Blickwinkel (35) aus gegenüberliegt, der zwischen der genannten ersten (210) und der genannten zweiten (220) Fläche der ersten (21) und der zweiten (22) Platte angeordnet ist.

3. Autonomes Gerät (10) gemäß Anspruch 1, **dadurch gekennzeichnet, dass** die erste Platte (21) geeignet ist, sich zu verformen, wenn ein Beutel (40) von dem elastischen Mechanismus (30) zwischen der ersten Fläche (210) der ersten Platte (21) und der zweiten Fläche (220) der zweiten Platte (22) derart mit Druck beaufschlagt ist, dass die erste Fläche (210) der ersten Platte (21) an jedem Punkt der ersten Fläche (210) der ersten Platte (21) konkav und gekrümmt ist, die der zweiten Fläche (220) der zweiten Platte (22) bei Vorhandensein eines Beutels (40) gegenüberliegt, der zwischen der ersten Fläche (210) der ersten Platte (21) und der zweiten Fläche (220) der zweiten Platte (22) und von einem Blickwinkel (35) mit Druck beaufschlagt ist, der zwischen der genannten ersten (210) und der genannten zweiten (220) Fläche der ersten (21) und der zweiten (22) Platte angeordnet ist.

4. Autonomes Gerät (10) gemäß Anspruch 3, **dadurch gekennzeichnet, dass** die erste Fläche (210) der ersten Platte (21) bei Fehlen des Beutels (40) und von einem Blickwinkel (35), der zwischen der genannten ersten (210) und der genannten zweiten (220) Fläche der ersten (21) und der zweiten (22) Platte angeordnete ist, eine konvexe Form aufweist.

5. Autonomes Gerät (10) gemäß irgendeinem der voranstehenden Ansprüche, **dadurch gekennzeichnet, dass** Überschneidungen von Schnittebenen (80) mit der zweiten Fläche (220) der zweiten Platte (22) Kurven des zweiten Grades in orthogonalen Bezugspunkten definieren, die in diesen Schnittebenen (80) angeordnet sind.

6. Autonomes Gerät (10) gemäß irgendeinem der voranstehenden Ansprüche, **dadurch gekennzeichnet, dass** Überschneidungen von Schnittebenen (80) mit der ersten Fläche (210) der ersten Platte (21) Kurven des zweiten Grades in orthogonalen Bezugspunkten definieren, die in diesen Schnittebenen (80) angeordnet sind.

7. Autonomes Gerät (10) gemäß irgendeinem der voranstehenden Ansprüche, **dadurch gekennzeichnet, dass** die genannte Konvexität der zweiten Fläche (220) der zweiten Platte (22) derart ist, dass diese zweite Fläche (220) der zweiten Platte (22) eine zwischen 2 und 3 mm inbegriffene Durchbiegung aufweist.

8. Autonomes Gerät (10) gemäß irgendeinem der Ansprüche 1 bis 6, **dadurch gekennzeichnet, dass** die Konvexität der zweiten Fläche (220) der zweiten Platte (22) derart ist, dass diese zweite Fläche (220) der zweiten Platte (22) eine zwischen 1 und 2 mm inbegriffene Durchbiegung, die bevorzugt gleich 1,5 mm beträgt, aufweist.

9. Autonomes Gerät (10) gemäß irgendeinem der voranstehenden Ansprüche, **dadurch gekennzeichnet, dass** die erste Platte (21) ein Deckel ist.

10. Autonomes Gerät (10) gemäß irgendeinem der voranstehenden Ansprüche, **dadurch gekennzeichnet, dass** es darüber hinaus ein einen Boden (251) umfassendes Gehäuse (25) umfasst.

11. Autonomes Gerät (10) gemäß dem voranstehenden Anspruch, **dadurch gekennzeichnet, dass** die zweite Platte (22) in Bezug auf den Boden (251) des Gehäuses (25) mobil ist und dass der genannte elastische Mechanismus (30) zwischen dem Boden (251) des Gehäuses (25) und der genannten zweiten Platte (22) angeordnet ist.

12. Autonomes Gerät (10) gemäß irgendeinem der voranstehenden Ansprüche, **dadurch gekennzeichnet, dass** die erste Platte (21) aus einem ein Plastikmaterial umfassenden Material gebildet ist.

13. Autonomes Gerät (10) gemäß irgendeinem der voranstehenden Ansprüche, **dadurch gekennzeichnet, dass** die erste Platte (21) aus einem Material gebildet ist, das mit Glasfasern geladenes Plastikmaterial umfasst.

14. Autonomes Gerät (10) gemäß irgendeinem der voranstehenden Ansprüche, **dadurch gekennzeichnet, dass** die zweite Platte (22) aus einem ein Plastikmaterial umfassenden Material gebildet ist.

15. Autonomes Gerät (10) gemäß irgendeinem der voranstehenden Ansprüche, **dadurch gekennzeichnet, dass** die zweite Fläche (220) der zweiten Platte (22) einen Nocken zum Befestigen des genannten Beutels (40) umfasst.

## Claims

1. Autonomous apparatus (10) adapted to receive an external bag (40) containing a fluid, and adapted to squeeze it in order to convey the said fluid from the said bag (40) to a living being, the said autonomous apparatus (10) comprising:
- a first plate (21) having a first surface (210),
- a second plate (22) having a second surface (220),
the said second surface (220) of the second plate (22) being convex from a point of view (35) located between said first (210) and second (220) surfaces of the said first (21) and second (22) plates, and the said second surface (220) of the second plate (22) is curved at any point of the said second surface (220) of the second plate (22) that faces the first surface (210) of the first plate (21).
the said autonomous apparatus (10) is configured:
- in a way that the said first surface (210) of the first plate (21) and the said second surface (220) of the second plate (22) are adapted to face one another, and
- in a way that allows for the said external bag (40) containing the fluid to be inserted between the said first (210) and second (220) surfaces of the said premier (21) and second (22) plates;
the said autonomous apparatus (10) additionally comprising an elastic mechanism (30):
- to bring the first (210) and second (220) surfaces of the first (21) and second (22) plates closer, and
- to put the said bag (40) under pressure between the first surface (210) of the first plate (21) and the second surface (220) of the second plate (22),
and wherein the first surface (210) is devoid of any opening at any point that faces the second surface (220), and wherein, either the first surface is concave and curved at any point of the first surface (210) that faces the second surface (220) in the absence of the bag (40) and from a point of view (35) located between the said first (210) and second (220) surfaces, or the first plate (21) is adapted to warp when a bag (40) is put under pressure by the elastic mechanism (30) between the first surface (210) and the second surface (220).

2. Autonomous apparatus (10) according to claim 1, **characterised in that** the said first surface (210) of the first plate (21) is concave and curved at any point of the first surface (210) of the first plate (21) that faces the second surface (220) of the second plate (22) in the absence of the bag (40), and from a point of view (35) located between the said first (210) and second (220) surfaces of the first (21) and second (22) plates.

3. Autonomous apparatus (10) according to claim 1, **characterised in that** the first plate (21) is adapted to warp while a bag (40) is put under pressure by the elastic mechanism (30) between the first surface (210) of the first plate (21) and the second surface (220) of the second plate (22) in a way that the said first surface (210) of the first plate (21) is concave and curved at any point of the first surface (210) of the first plate (21) that faces the second surface (220) of the second plate (22) in the presence of a bag (40) put under pressure between the first surface (210) of the first plate (21) and the second surface (220) of the second plate (22) and from a point of view (35) located between the said first (210) and second (220) surfaces of the first (21) and second (22) plates.

4. Autonomous apparatus (10) according to claim 3, **characterised in that** the first surface (210) of the first plate (21) has a convex shape in the absence of a bag (40) and from a point of view (35) located between the said first (210) and second (220) surfaces of the first (21) and second (22) plates.

5. Autonomous apparatus (10) according to any one of the preceding claims, **characterised in that** intersections of section planes (80) with the second surface (220) of the second plate (22) define second-degree curves in orthogonal coordinate systems located in these section planes (80).

6. Autonomous apparatus (10) according to any one of the preceding claims, **characterised in that** intersections of section planes (80) with the first surface (210) of the first plate (21) define second-degree curves of orthogonal coordinate systems enclosed in these section planes (80).

7. Autonomous apparatus (10) according to any one of the preceding claims, **characterised in that** the said convexity of the second surface (220) of the second plate (22) is such that this second surface (220) of the second plate (22) has a deflection between 2 and 3 mm.

8. Autonomous apparatus (10) according to any one of the claims from 1 to 6, **characterised in that** the convexity of the second surface (220) of the second plate (22) is such that this second surface (220) of the second plate (22) has a deflection between 1 and 2 mm, preferably equal to 1.5 mm.

9. Autonomous apparatus (10) according to any one of the preceding claims, **characterised in that** the first plate (21) is a cover plate.

10. Autonomous apparatus (10) according to any one of the preceding claims, **characterised in that** it additionally has an enclosure (25) comprising a base (251).

11. Autonomous apparatus (10) according to the preceding claim, **characterised in that** the second plate (22) is movable in line with the base (251) of the enclosure (25), and **in that** the said elastic mechanism (30) is set in between the base (251) of the enclosure (25) and the said second plate (22).

12. Autonomous apparatus (10) according to any one of the preceding claims, **characterised in that** the first plate (21) consists of a material comprising a plastic substance.

13. Autonomous apparatus (10) according to any one of the preceding claims, **characterised in that** the first plate (21) consists of a material comprising a plastic substance reinforced with fiberglass.

14. Autonomous apparatus (10) according to any one of the preceding claims, **characterised in that** the second plate (22) consists of a material comprising a plastic substance.

15. Autonomous apparatus (10) according to any one of the preceding claims, **characterised in that** the second surface (220) of the second plate (22) comprises an ergot for fastening the said bag (40) .
